(19) Europäisches Patentamt
European Patent Office
Office européen des brevets

(11) EP 0 976 397 B1

(12) **EUROPÄISCHE PATENTSCHRIFT**

(45) Veröffentlichungstag und Bekanntmachung des Hinweises auf die Patenterteilung:
**15.12.2004 Patentblatt 2004/51**

(51) Int Cl.⁷: **A61K 31/19**, A61K 9/70

(21) Anmeldenummer: **99113743.1**

(22) Anmeldetag: **14.07.1999**

(54) **Verbesserte Freisetzung von Ibuprofen aus Heissschmelzklebemassen durch Zusatz von pharmazeutischen Hilfsstoffen**

Improved release of ibuprofen from pressure-sensitive hot melt adhesives by the addition of pharmaceutical excipients

Amélioration de la libération d'ibuprofène depuis des adhésifs thermofusibles par addition d'excipients pharmaceutiques

(84) Benannte Vertragsstaaten:
**AT BE CH CY DE DK ES FI FR GB GR IE IT LI LU MC NL PT SE**

(30) Priorität: **31.07.1998 DE 19834496**

(43) Veröffentlichungstag der Anmeldung:
**02.02.2000 Patentblatt 2000/05**

(73) Patentinhaber: **Beiersdorf Aktiengesellschaft**
**20245 Hamburg (DE)**

(72) Erfinder:
- **Mayan, Robert, Dr.**
  **21614 Buxtehude (DE)**
- **Wasner, Matthias**
  **21029 Hamburg (DE)**
- **Philipp, Peter, Dr.**
  **22885 Barsbüttel (DE)**

(56) Entgegenhaltungen:
**EP-A- 0 305 756**
**EP-A- 0 360 467**
**EP-A- 0 617 972**
**DE-A- 4 403 487**
**US-A- 5 352 722**

- **PATENT ABSTRACTS OF JAPAN vol. 1996, no. 01, 31. Januar 1996 (1996-01-31) & JP 07 233050 A (SEKISUI CHEM CO LTD), 5. September 1995 (1995-09-05)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die verbesserte Freisetzung von Wirkstoffen aus Heißschmelzklebemassen durch den Zusatz von pharmazeutischen Hilfsstoffen.

Bei der Formulierung von Arzneimitteln zur topischen Applikation von Wirkstoffen kommt der Wahl der Hilfsstoffe entscheidende Bedeutung zu.

So ist bekannt, daß ein Zusammenhang zwischen Löslichkeit eines Wirkstoffs in einem Vehikel und seiner Freisetzung aus diesem Vehikel besteht. Wird ein wirkstoffhaltiges Vehikel auf die Haut appliziert, dann stellt sich ein Verteilungsgleichgewicht des Wirkstoffs zwischen Vehikel und Haut ein. Die Lage dieses Gleichgewichts wird von der Löslichkeit in beiden Phasen bestimmt und durch den Verteilungskoeffizienten $P_s$ beschrieben.

Nach dem 1. Fick'schen Gesetz gilt für den Flux eines gelösten Stoffes aus einem Vehikel in die Haut

$$J_s = \frac{P_s\, C_i\, D_i}{h}$$

wobei $J_s$ den Flux, $P_s$ den Verteilungskoeffizienten, $C_i$ die Konzentration des Wirkstoffs im Vehikel, $D_i$ den Diffusionskoeffizienten und h die Dicke der Haut bezeichnet.

Für die Löslichkeit eines Polymers in einem Solvens gilt nach Hildebrand und Scott folgende Gleichung:

$$\Delta H_m = V_m\ \phi_1\phi_2(\delta_1\text{-}\delta_2)^2$$

mit $\Delta H_m$ Mischungsenthalpie, $V_m$ totales Volumen der Mischung, $\Phi$ Volumenanteil der Komponenten und $\delta$ Löslichkeitsparameter,

wobei gute Löslichkeit gegeben ist, wenn die Parameter möglichst gleich sind und damit der Term ($\delta_1$ - $\delta_2$) klein wird. Eine Differenz von $\delta_1$ - $\delta_2$ < 2 gibt den Hinweis, daß man Löslichkeit erwarten kann.

Der Hildebrand'sche Löslichkeitsparameter ist definiert als die Summe aller zwischenmolekularen Anziehungskräfte eines Stoffes und kann durch verschiedene Methoden ermittelt werden. Eine allgemeine Diskussion des Konzepts der Löslichkeitsparameter ist in einer Reihe von Publikationen nachzulesen; beispielhaft sei hier Vaughan, C.D., Journal of the Society of Cosmetic Chemists **36**, 319-333 (1985) genannt.

Aus diesen Gleichungen kann nach Sloan, K.B. et al, Journal of Pharmaceutical Sciences, 75, 744 (1986), folgender Ausdruck für das Verteilungsgleichgewicht eines Wirkstoffs zwischen Vehikel und Haut abgeleitet werden:

$$\ln P_s = \frac{(\delta_i\text{-}\delta_v)^2 V_i \phi_v^2}{RT} - \frac{(\delta_i\ \delta_s)^2 V_i \phi_s^2}{RT} = \ln\ \frac{C_i^{skin}}{C_i^{vehicle}}$$

- $V_i$ molares Volumen des Wirkstoffs
- $\delta_i$ Löslichkeitsparameter Wirkstoff
- $\delta_v$ Löslichkeitsparameter Vehikel
- $\delta_s$ Löslichkeitsparameter Haut

Die Freisetzung eines niedermolekularen Stoffes aus einer polymeren Matrix wird bestimmt durch ein komplexes Zusammenspiel von spezifischen Wechselwirkungen aller Komponenten. Gemäß obiger Gleichung wird die Konzentration des Wirkstoffs in der Haut hoch, wenn die Löslichkeit in der Haut hoch und die Löslichkeit im Vehikel niedrig ist. Eine Variation der Freisetzung eines Wirkstoffs aus einem Vehikel ist folglich durch Anpassung des Löslichkeitsparameters $\delta_v$ dieses Vehikels möglich.

Da der Hildebrand'sche Löslichkeitsparameter $\delta$ eine Stoffkonstante ist, folgt weiterhin aus dieser Gleichung, daß für jeden Wirkstoff eine spezifische Modifikation des Vehikels notwendig ist. Analogieschlüsse innerhalb einer chemischen Stoffgruppe oder Wirkstoff-Familie sind aufgrund signifikanter Unterschiede der 8-Werte nicht möglich. Dies verdeutlichen beispielhaft Werte für chemisch eng verwandte Hilfsstoffe wie Cetylalkohol ($\delta$ = 8.94), Laurylalkohol ($\delta$ = 9.51) und Caprylalkohol ($\delta$ = 10.09) oder auch Ethylenglycol ($\delta$ = 14.50), Diethylenglycol ($\delta$ = 13.61) und Triethylenglycol ($\delta$ = 12.21).

Wie unter anderem aus US 4,555,524 ersichtlich, ist für topische Darreichungsformen wie Cremes oder Salben die nutzbringende Verwendung von Hilfsstoffen zur deutlich verbesserten Freisetzung eines Wirkstoffs bekannt. Dort wird durch Lösen von Ibuprofen in einer Kombination von pharmazeutischen Hilfsstoffen die erhöhte Freisetzung des Wirkstoffs gegenüber Formulierungen erzielt, die den Wirkstoff ungelöst enthalten. Eine gezielte Einstellung des

Löslichkeitsparameters δ, des Vehikels zur optimalen Freisetzung von Ibuprofen wird hier nicht beschrieben. Die eingesetzten Mengen an Hilfsstoff sind außerdem so groß, daß klebende Darreichungsformen damit nicht hergestellt werden können.

Bei sogenannten drug-in-adhesive Systemen, also bei wirkstoffhaltigen Pflastern, die den Arzneistoff in der Klebemasse enthalten, wird die oben beschriebene Strategie nur in Einzelfällen angewandt.

Heißschmelzklebemassen erlauben die Vermeidung von Nachteilen, die mit dem bei herkömmlichen wirkstoffhaltigen Klebemassen notwendigen Einsatz von Lösemitteln verbunden sind. Zu erwähnen sind hier die Schädlichkeit der meisten organischen Lösungsmittel, hohe technische Aufwände für Absaugung und Wiedergewinnung, hohe Kosten für erforderliche hochreine Lösungsmittel und besonders ein sehr hoher Aufwand zur Entfernung von Lösungsmittelresten aus der Matrix.

Die Vorteile der Heißschmelzklebemassen bei der Herstellung von wirkstoffhaltigen Klebemassen werden schon seit einigen Jahren in der Patentliteratur beschrieben (siehe zum Beispiel EP 0 305 757 A1).

Die zielgerichtete Optimierung der Löslichkeit eines Wirkstoffs in einer Matrix ist für Heißschmelzklebemassen auf Polystyrol-Blockcopolymer-Basis nicht bekannt.

US 5,527,536 beschreibt wirkstoffhaltige Heißschmelzklebemassen auf Polystyrol-Blockcopolymer-Basis, besonders Polystyrolblock-copoly(ethylenbutylen)-block-Polystyrol (SEBS). Diese Massen enthalten neben SEBS Klebrigmacher und Alterungsschutzmittel. Einsatz und Nutzen von zugesetzten pharmazeutischen Hilfsstoffen wird nicht beschrieben.

Für Heißschmelzklebemassen auf Basis von Ethylen-Vinylacetat-Copolymeren wird die Optimierung der Freisetzung von Wirkstoffen in US 4,837,025 beschrieben. Verwendung finden hier Fettsäureester von Polyalkoholen.

WO 93/00058 beschreibt die Optimierung von wirkstoffhaltigen Klebemassen unter Anwendung der oben geschilderten Prinzipien, wobei die Variation des Löslichkeitsparameters $\delta_v$ durch Mischen zweier polymerer Klebemassen mit unterschiedlichen Löslichkeitsparametem erzielt wird, bevorzugt einer Silicon- und einer Acrylat-Klebemasse. Niedermolekulare Hilfsstoffe werden zur Anpassung des $\delta_v$ nicht eingesetzt.

US 5,702,720 beschreibt ein wirkstoffhaltiges Pflaster, das Flurbiprofen, gelöst in einer Acrylat-Klebemasse, enthält. Ebenfalls enthaltenes Isopropylmyristat dient als Beschleuniger, der die Penetration des Wirkstoffs durch die Haut verstärkt. Dieser niedermolekulare Hilfsstoff wird offensichtlich nicht zur Optimierung der Löslichkeit im Vehikel eingesetzt.

WO 94/23713 beschreibt entzündungshemmende Arzneimittel auf Phenylpropionsäure- und Phenylessigsäure-Basis zur topischen Applikation, welche Kombinationen von lipophilen und hydrophilen Hilfsstoffen enthalten. Über Wirkungsweise und Funktion dieser Hilfsstoffe werden keine Angaben gemacht.

Eine Anpassung des Löslichkeitsparameters $\delta_v$ des Vehikels durch Zugabe von lipophilen und hydrophilen Hilfsstoffen ist offensichtlich nicht beabsichtigt, wäre sie doch kompliziert und umständlich. Zudem würden die Auswirkungen der unterschiedlichen Hilfsstoffe auf $\delta_v$ gegenläufig sein und sich aufheben.

Gemäß EP 0 827 741 A2 kann die Freisetzung von Ketoprofen aus klebenden Vehikeln durch Zusatz von Hilfsstoffen verbessert werden. Beispielhaft werden in Hexan/Toluol gelöste Polyisobutylene als Gerüst für die Klebemasse beschrieben.

EP 617972 A2 offenbart ein dermales therapeutisches System umfassend ein Trägermaterial, Heißschmelzklebemasse und einen pharmazeutischen Wirkstoff, der Ibuprofen sein kann. Erwähnt wird, dass mit Hilfe von Hilfsstoffen oder penetrationsfördemden Substanzen therapiegerechte Freigabeprofile zu erreichen sind

DE 4403487 A1 offenbart transdermale therapeutische Systeme, bei denen ein Wirkstoff, beispielsweise Ibuprofen, in einem Acrylat-Haftschmelzklebstoff eingearbeitet ist.

Aus EP 360467 A2 und US 5352722 ist bekannt, durch Zugabe von Hilfsstoffen die Freisetzung von Wirkstoffen aus Heißschmelzklebemassen zu verbessern.

Bei Ibuprofen handelt es sich um die (±)-2-(4-Isobutylphenyl)propionsäure ($C_{13}H_{18}O_2$; $M_R$ 206,28; Schmelzpunkt 75 bis 77 °C) mit der allgemeinen Formel

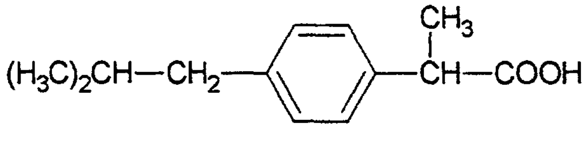

(nach Römpp Lexikon Chemie, Version 1.3, Stuttgart/New York: Georg Thieme Verlag 1997). Ibuprofen ist ein bekannter Wirkstoff mit analgetischen und entzündungshemmenden Eigenschaften und wird deshalb bei Weichteil-Rheuma und entzündlichen Gelenkserkrankungen eingesetzt. Meist wird der Wirkstoff in Form von Tabletten oder Suppositorien verwendet. Orale Verabreichung kann jedoch zu Nebenwirkungen wie Übelkeit, Erbrechen, Schwindelgefühl und Kopfschmerzen führen. Ibuprofen darf aus diesem Grund nicht von Patienten mit Magengeschwüren eingenommen werden. Nach topischer Applikation erreicht der Wirkstoff das Ziel ohne vorherige Metabolisierung durch die Leber. Dadurch lassen sich Nachteile wie der sog. first-pass effect und Nebenwirkungen vermeiden, die mit der oralen oder rektalen Gabe von Ibuprofen verbunden sind. Die Freisetzung des Wirkstoffs aus einer klebenden Matrix hat gegenüber herkömmlichen topischen Darreichungsformen wie Cremes oder Salben weitere Vorteile. Der Wirkstoff wird kontinuierlich aus der Matrix freigesetzt und nicht in einzelnen Dosen appliziert. Beim Auftreten von unerwünschten Nebenwirkungen kann das wirkstoffhaltige Pflaster einfach entfernt und damit die Abgabe von Ibuprofen unterbrochen werden.

Aufgabe der Erfindung ist es, die aus dem Stand der Technik bekannten Nachteile zu vermeiden und somit die Freisetzung von Ibuprofen aus Heißschmelzklebemassen zu verbessern.

Gelöst wird diese Aufgabe durch ein wirkstoffhaltiges Pflaster enthaltend ein Trägermaterial und eine darauf zumindest partiell aufgebrachten Heißschmelzklebemasse, wobei die Heißschmelzklebemasse Ibuprofen sowie Hilfsstoffe enthält. Die Hilfsstoffe werden gewählt aus der Gruppe

a.) der Ester gebildet aus Fettsäuren mit 8 bis 18 Kohlenstoffatomen und kurzkettigen Alkoholen oder Fettalkoholen und/oder Ester oder Ether gebildet aus Polyethylenglykol der Formel

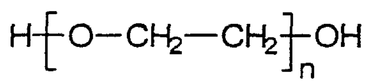

mit n= 6 bis 12 und Fettalkoholen mit 8 bis 18 Kohlenstoffatomen und/oder

b.) der Propylenglycol-mono-, di-ester, Gylcerinmono-, -di- und -triester mit Fettalkoholen mit 8 bis 18 Kohlenstoffatomen, Fettalkoholen mit 8 bis 18 Kohlenstoffatomen, Propylenglykol und Polyethylenglykol.

Bei Fettalkoholen handelt es sich um eine Sammelbezeichnung für die durch Reduktion der Triglyceride, Fettsäuren bzw. Fettsäuremethylester erhältlichen linearen, gesättigten oder ungesättigten primären Alkohole (1-Alkanole) mit 6 bis 22 Kohlenstoff-Atomen.

Fettalkohole stellen neutrale, farblose, hochsiedende, ölige Flüssigkeiten oder weiche farblose Massen dar, die in Wasser schwer- bis unlöslich, in Alkohol und Ether hingegen leicht löslich sind.

In der anschließenden Tabelle sind physikalisch-chemische Daten der Fettalkohole angegeben (aus Römpp Lexikon Chemie, Version 1.3, Stuttgart/New York: Georg Thieme Verlag 1997)

Tabelle:

| Physikalisch-chemische Daten der Fettalkohole. | | | | |
|---|---|---|---|---|
| Alkohol | Formel | $M_R$ | Schmp. °C | Sdp. °C/ kPa |
| 1-Hexanol (Capronalkohol) | $C_6H_{14}O$ | 102,18 | -51,6 | 157,2 |
| 1-Heptanol (Önanthalkohol) | $C_7H_{16}O$ | 116,20 | -30,0 | 177 |
| 1-Octanol (Caprylalkohol) | $C_8H_{18}O$ | 130,23 | -16,3 | 194,5 |
| 1-Nonanol (Pelargonalkohol) | $C_9H_{20}O$ | 144,26 | | 212 |
| 1-Decanol (Caprinalkohol) | $C_{10}H_{22}O$ | 158,28 | -7,0 | 229 |
| 1-Undecanol | $C_{11}H_{24}O$ | 172,31 | 16,3 | 131/2,0 |
| 10-Undecen-1-ol | $C_{11}H_{22}O$ | 170,30 | -2 | 133/2,1 |
| 1-Dodecanol (Laurylalkohol) | $C_{12}H_{26}O$ | 186,34 | 23,8 | 150/2,7 |
| 1-Tridecanol | $C_{13}H_{28}O$ | 200,36 | | 155/2,0 |
| 1-Tetradecanol (Myristylalkohol) | $C_{14}H_{30}O$ | 214,39 | 38,0 | 167/2,0 |
| 1-Pentadecanol | $C_{15}H_{32}O$ | 228,42 | 44,0 | |

Tabelle: (fortgesetzt)

| Physikalisch-chemische Daten der Fettalkohole. | | | | |
|---|---|---|---|---|
| Alkohol | Formel | $M_R$ | Schmp. °C | Sdp. °C/ kPa |
| 1-Hexadecanol (Cetylalkohol) | $C_{16}H_{34}O$ | 242,45 | 49,3 | 190/2,0 |
| 1-Heptadecanol | $C_{17}H_{36}O$ | 256,47 | 54,0 | 308 |
| 1-Octadecanol (Stearylalkohol) | $C_{18}H_{38}O$ | 270,50 | 59,0 | 210/2,0 |
| 9-*cis*-Octadecen-1-ol (Oleylalkohol) | $C_{18}H_{36}O$ | 268,48 | -7,5 | 209/2,0 |
| 9-*trans* Octadecen-1-ol (Elaidylalkohol) | $C_{18}H_{36}O$ | 268,48 | 36,5 | 216/2,4 |
| 9-*cis*-Octadecen-1,12-diol (Ricinolalkohol) | $C_{18}H_{36}O_2$ | 284,48 | | 182/0,07 |
| *all*-*cis*-9,12-Octaclecadien-1-ol (Linoleylalkohol) | $C_{18}H_{34}O$ | 266,47 | -5 | 153/0,4 |
| *all*-*cis*-9,12,15-Octadecatrien-1-ol (Linolenylalkohol) | $C_{18}H_{32}O$ | 264,45 | | 133/0,3 |
| 1-Nonadecanol | $C_{19}H_{40}O$ | 284,53 | 62 | 167/0,04 |
| 1-Eicosanol (Arachidylalkohol) | $C_{20}H_{42}O$ | 298,55 | 65,5 | 220/0,4 |
| 9-*cis*-Eicosen-1-ol (Gadoleylalkohol) | $C_{20}H_{40}O$ | 296,54 | | 209/2,0 |
| 5,8,11,14-Eicosatetraen-1-ol | $C_{20}H_{34}O$ | 290,49 | | |
| 1-Heneicosanol | $C_{21}H_{44}O$ | 312,58 | 69,5 | |
| 1-Docosanol (Behenylalkohol) | $C_{22}H_{46}O$ | 326,61 | 73,5 | 180/0,03 |
| 1-3-*cis*-Docosen-1-ol (Erucylalkohol) | $C_{22}H_{44}O$ | 324,59 | 34,5 | 241/1,3 |
| 1-3-*trans*-Docosen-1-ol (Brassidylalkohol) | $C_{22}H_{44}O$ | 324,59 | 53,5 | 241/1,1 |

Unter Polyethylenglykolen werden zur Klasse der Polyether gehörende Polyalkylenglykole der allgemeinen Formel:

$$H\left[O-CH_2-CH_2\right]_n OH$$

verstanden.

Polyethylenglykole werden technisch hergestellt durch eine basische katalysierte Polyaddition von Ethylenoxid (Oxiran) in meist geringe Mengen Wasser enthaltenden Systemen mit Ethylenglykol als Startmolekül. Sie haben Molmassen im Bereich von ca. 200-5 000 000 g/mol, entsprechend Polymerisationsgraden n von ca. 5 bis >100 000.

Vorzugsweise sind die Hilfsstoffe in der Heißschmelzklebemasse zu 1 bis 20 Gew.-% enthalten, insbesondere zu 2 bis 15 Gew.-%.

Die Heißschmelzklebemasse ist in einer weiteren bevorzugten Ausführungsform eine Polystyrol-Blockcopolymer-Heißschmelzklebemasse, ganz besonders bevorzugt eine Polystyrolblock-copoly(ethylenbutylen)-block-Polystyrol-Heißschmelzklebemasse.

Als Heißschmelzklebemassen lassen sich vorteilhafterweise thermoplastische Heißschmelzselbstklebemassen auf Basis natürlicher und synthetischer Kautschuke und anderer synthetischer Polymere wie Acrylate, Methacrylate, Polyurethane, Polyolefine, Polyvinylderivate, Polyester oder Silikone einsetzen. Zusatzstoffe wie Klebharze, Weichmacher, Stabilisatoren und andere Hilfsstoffe können soweit erforderlich zugesetzt werden.

Der Erweichungspunkt der Heißschmelzklebemassen sollte höher als 50 °C liegen, da die Applikationstemperatur in der Regel mindestens 90 °C beträgt, bevorzugt zwischen 120 °C und 150 °C beziehungsweise 180 °C und 240 °C bei Silikonen.

Insbesondere Heißschmelzselbstklebemassen auf Basis von Blockcopolymeren zeichnen sich durch ihre vielfältigen Variationsmöglichkeiten aus, denn durch die gezielte Auswahl der Klebrigmacher, der Weichmacher sowie des Polymerisationsgrads und der Molekulargewichtsverteilung der Einsatzkomponenten wird die notwendige funktionsgerechte Verklebung mit der Haut auch an kritischen Stellen des menschlichen Bewegungsapparates gewährleistet.

Die hohe Scherfestigkeit der Heißschmelzselbstklebemasse auf Blockcopolymer-Basis wird durch die hohe Kohäsivität des Polymeren erreicht. Die gute Anfaßklebrigkeit ergibt sich durch die eingesetzte Palette an Klebrigmachern und Weichmachern.

Für besonders starkklebende Systeme basiert die Heißschmelzselbstklebemasse bevorzugt auf Blockcopolymeren, insbesondere A-B-, A-B-A-Blockcopolymere oder deren Mischungen. Phase A ist vornehmlich Polystyrol oder dessen Derivate, Phase B enthält Ethylen, Propylen, Butylen, Butadien, Isopren oder deren Mischungen, hierbei besonders bevorzugt Ethylen und Butylen oder deren Mischungen.
Beide Phasen A und B sind nicht mischbar, so daß sich im festen Zustand Domänen aus Polystyrol- und Copoly (ethylenbutylen)-Blöcken bilden.

Polystyrol befindet sich unterhalb der Glasübergangstemperatur und trägt damit zur hohen Scherfestigkeit bei, während sich Copoly(ethylenbutylen)-Blöcke über ihrer Glasübergangstemperatur befinden und elastisches Verhalten zeigen.

Die Glasübergangstemperatur ist die Temperatur, bei der amorphe oder teilkristalline Polymere vom flüssigen oder gummielastischen Zustand in den hartelastischen oder glasigen Zustand übergehen oder umgekehrt (Römpp Chemie-Lexikon, 9. Aufl., Band 2, Seite 1587, Georg Thieme Verlag Stuttgart - New York, 1990). Er entspricht dem Maximum der Temperaturfunktion bei vorgegebener Frequenz.
Besonders für medizinische Anwendungen ist ein relativ niedriger Glasübergangspunkt notwendig.

Insbesondere die gezielte Abmischung von Di-Block- und Tri-Blockcopolymeren ist vorteilhaft, wobei ein Anteil an Di-Blockcopolymeren von kleiner 80 Gew.-% bevorzugt wird.

In einer vorteilhaften Ausführung weist die Heißschmelzselbstklebemasse die nachfolgend angegebene Zusammensetzung auf:

| | |
|---|---|
| 10 Gew.-% bis 90 Gew.-% | Blockcopolymere, |
| 5 Gew.-% bis 80 Gew.-% | Klebrigmacher wie Öle, Wachse, Harze und/oder deren Mischungen, bevorzugt Mischungen aus Harzen und Ölen, |
| weniger als 60 Gew.-% | Weichmacher, |
| weniger als 15 Gew.-% | Additive, |
| weniger als 5 Gew.-% | Stabilisatoren, |
| 0,01 Gew.-% bis 10 Gew.-% | Wirkstoff oder Wirkstoffe. |

Die als Klebrigmacher dienenden aliphatischen oder aromatischen Öle, Wachse und Harze sind bevorzugt Kohlenwasserstofföle, -wachse und -harze, wobei sich die Öle, wie Paraffinkohlenwasserstofföle, oder die Wachse, wie Paraffinkohlenwasserstoffwachse, durch ihre Konsistenz günstig auf die Hautverklebung auswirken. Diese Zusätze dienen dabei der Einstellung der Klebeeigenschaften und der Stabilität. Gegebenenfalls kommen weitere Stabilisatoren und andere Hilfsstoffe zum Einsatz.

Ein Füllen der Klebemasse mit mineralischen Füllstoffen, Fasern, Mikrohohl- oder -vollkugeln ist möglich.

Die Heißschmelzselbstklebemasse weist einen Erweichungspunkt von größer als 50 °C auf, bevorzugt 70 °C bis 220 °C, ganz besonders bevorzugt 75 °C bis 140 °C.

Die Heißschmelzselbstklebemassen sind vorzugsweise so eingestellt, daß sie bei einer Frequenz von 0,1 rad/s eine dynamisch-komplexe Glasübergangstemperatur von weniger als 10 °C, bevorzugt von 0 °C bis -30 °C ganz besonders bevorzugt von -6 °C bis -25 °C, aufweisen.

Insbesondere an Pflaster werden hohe Anforderungen bezüglich der Klebeeigenschaften gestellt. Für eine ideale Anwendung sollte die Heißschmelzselbstklebemasse eine hohe Anfaßklebrigkeit besitzen. Die funktionsangepaßte Klebkraft auf der Haut sollte vorhanden sein. Weiterhin ist eine hohe Scherfestigkeit der Heißschmelzselbstklebemasse notwendig, damit beim Ablösen des Pflasters keine Masserückstände auf der Haut verbleiben. Werden größere Mengen der pharmazeutischen Hilfsstoffe zugesetzt, kann durch Reduzierung oder Eliminierung der Weichmacher die hohe Scherfestigkeit erhalten werden.

Die Produkteigenschaften wie Anfaßklebrigkeit, Glasübergangstemperatur und Scherstabilität lassen sich mit Hilfe einer dynamisch-mechanischen Frequenzmessung gut quantifizieren. Hierbei wird ein schubspannungsgesteuertes Rheometer verwendet. Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur die Heißschmelzselbstklebemasse zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient (Q = tan δ) zwischen dem Verlustmodul (G'' viskoser Anteil) und dem Speichermodul (G' elastischer Anteil) ermittelt.

$$Q = \tan \delta = G''/G'$$

Für das subjektive Empfinden der Anfaßklebrigkeit (Tack) wird eine hohe Frequenz gewählt sowie für die Scherfestigkeit eine niedrige Frequenz.

Eine hoher Zahlenwert bedeutet eine bessere Anfaßklebrigkeit und eine schlechtere Scherstabilität.

Bevorzugt werden erfindungsgemäß Heißschmelzselbstklebemassen, bei denen das Verhältnis des viskosen Anteils zum elastischen Anteil bei einer Frequenz von 100 rad/s bei 25 °C größer 0,7 ist, bevorzugt 1,0 bis 5,0, oder Heißschmelzselbstklebemassen, bei denen das Verhältnis des viskosen Anteils zum elastischen Anteil bei einer Frequenz von 0,1 rad/s bei 25 °C kleiner 0,6 ist, bevorzugt zwischen 0,4 und 0,02, ganz besonders bevorzugt zwischen 0,3 und 0,1.

Um die funktionsgerechte Verwendung sicherzustellen, können die Heißschmelzklebemassen geschäumt sein, und zwar vorzugsweise geschäumt mit inerten Gasen wie Stickstoff, Kohlendioxid, Edelgasen, Kohlenwasserstoffen oder Luft oder deren Gemischen. In manchen Fällen hat sich ein Aufschäumen zusätzlich durch thermische Zersetzung gasentwickelnder Substanzen wie Azo-, Carbonat- und Hydrazid-Verbindungen als geeignet erwiesen.

Der Schäumungsgrad, d.h. der Gasanteil, sollte mindestens etwa 5 Vol.-% betragen und kann bis zu etwa 85 Vol.-% reichen. In der Praxis haben sich Werte von 10 Vol.-% bis 75 Vol.-%, bevorzugt 50 Vol.-%, gut bewährt. Wird bei relativ hohen Temperaturen von ungefähr 100 °C und vergleichsweise hohem Innendruck gearbeitet, entstehen sehr offenporige Klebstoffschaumschichten, die besonders gut luft- und wasserdampfdurchlässig sind.

Weiterhin umfaßt der Erfindungsgedanke wirkstoffhaltige Pflaster mit einem Trägermaterial und einer darauf zumindest partiell aufgebrachten Heißschmelzklebemasse, die sich dadurch auszeichnen, daß die Heißschmelzklebemasse Ibuprofen enthält.

Vorzugsweise liegen die Mengenkonzentrationen des Ibuprofen in der Heißschmelzklebemasse zwischen 0,01 bis 50 Gew.-%, vorzugsweise 0,1 bis 20 Gew.-%.

Als Trägermaterialien für die Heißschmelzklebemassen eignen sich alle starren und elastischen Flächengebilde aus synthetischen und natürlichen Rohstoffen. Bevorzugt sind Trägermaterialien die nach Applikation der Klebemasse so eingesetzt werden können, daß sie Eigenschaften eines funktionsgerechten Verbandes erfüllen. Beispielhaft sind Träger wie Gewebe, Gewirke, Gelege, Vliese, Laminate, Netze, Folien, Schäume und Papiere aufgeführt. Weiter können diese Materialien vor- beziehungsweise nachbehandelt werden. Gängige Vorbehandlungen sind Corona und Hydrophobieren; geläufige Nachbehandlungen sind Kalandern, Tempern, Kaschieren, Stanzen und Eindecken.

Schließlich kann das Pflaster nach dem Beschichtungsvorgang mit einem klebstoffabweisenden Trägermaterial, wie silikonisierte Polyesterfolie, eingedeckt oder mit einer Wundauflage oder einer Polsterung versehen werden. Anschließend werden die Pflaster in der gewünschten Größe ausgestanzt.

Das erfindungsgemäße Pflaster weist eine Klebkraft auf Stahl von mindestens 1,0 N/cm auf, bevorzugt eine Klebkraft zwischen 1,5 N/cm und 6,0 N/cm, ganz besonders bevorzugt eine Klebkraft zwischen 1,5 N/cm und 6,0 N/cm. Auf anderen Untergründen können höhere oder niedrigere Klebkräfte erreicht werden.

Die erfindungsgemäß als Hilfsstoffe eingesetzten Substanzen finden vielseitige Anwendung in pharmazeutischen und kosmetischen Produkten sowie Nahrungsmitteln und zeichnen sich durch hervorragende, nachgewiesene Verträglichkeit aus.

Überraschenderweise ist eine Verarbeitung der beschriebenen pharmazeutischen Hilfsstoffe in Heißschmelzklebemassen ohne signifikante Verringerung der Scherfestigkeit möglich. Weiterhin erlauben die geprüften Hilfsstoffe eine große Verbesserung der Freisetzung.

Im folgenden soll die Erfindung anhand mehrerer Beispiele dargestellt werden.

**Beispiele**

**Herstellung von Ibuprofen haltigen SEBS-Klebemassen unter Zusatz von Hilfsstoffen**

Als wirkstofffreie Klebmasse dient die SEBS-Klebmasse mit der Bezeichnung E94 der HB Fuller GmbH, D-21335 Lüneburg. Der Wirkstoff Ibuprofen wird in Ethyloleat bei 60 °C gelöst und der in einem Thermokneter aufgeschmolzenen Klebmasse zugeführt. Die Einsatzkomponenten werden bei einer Temperatur von 90 °C über einem

Zeitraum von 3h homogenisiert.
Die so hergestellte Klebmasse wird aufgeschmolzen einer Beschichtungseinheit zugeführt und auf silikonisiertes Papier mit einem Masseauftrag von 300g/$m^2$ aufgetragen. Das Trägermaterial wird anschließend zukaschiert.

**Bestimmung der Freisetzung von Wirkstoff auf Schweinehaut**

Die hergestellten, wirkstoffhaltigen Klebemassen wurden wie folgt auf Freisetzung des Wirkstoffs geprüft: Die Freisetzung wurde an exzidierter Schweinehaut (Hornschicht, Epidermis, Dermis) bestimmt. Dazu wurden Pflasterstücke geeigneter Größe auf die Hornschicht geklebt. Die Haut war während des Versuchs dermisseitig von einer Rezeptorphase unterspült. Nach der vorgesehenen Zeit wurden die Pflasterstücke entfernt und der verbliebene Wirkstoffgehalt bestimmt. Dazu wurde die Klebmasse aufgelöst und die Lösung mittels HPLC analysiert. Ebenso wurden nicht applizierte Pflasterstücke untersucht. Die Freisetzung ergibt sich aus der Differenz der Wirkstoffgehalte.
Die erhaltenen Freisetzungsergebnisse für Ibuprofen weisen die Wirkung der zugesetzten Hilfsstoffe nach. Im Falle der beispielhaft untersuchten SEBS-Klebemasse konnte die Freisetzung von nicht meßbar (ohne Zusatz von Hilfsstoff) bis auf 185 µg/$cm^2$ 24h (durch Zusatz von Ethyloleat) gesteigert werden. Weitere Ergebnisse sind Tabelle 1 zu entnehmen.

Tabelle 1:

| Freisetzung von Ibuprofen aus SEBS-Heißschmelzklebemassen | | |
|---|---|---|
| **Verwendete Klebemasse** | **Pharmazeutischer Hilfsstoff Freisetzung Schweinehaut** | |
| | | µg/$cm^2$*24h |
| SEBS E94 | - | nicht meßbar |
| SEBS E94 | Ethyloleat (5%) | 185 |
| SEBS E94 | 2-Octyldodecylmyristat (5%) | 116 |
| SEBS E94 | Cetostearyl-isononanoat (5%) | 102 |
| SEBS E94 | Cetylpalmitat (5%) | 155 |
| SEBS E94 | Polyethylenglykol(400)monostearat (5%) | 140 |
| SEBS E94 | Polyethylenglykol 6000 (5%) | 46 |
| SEBS E94 | Cocoglyceride (5%) | 134 |
| SEBS E94 | Polyethylenglykol 400 (5%) | 103 |
| SEBS E94 | 1-Octadecanol (5%) | 140 |
| SEBS E94 | Glycerinmonooleat (5%) | 100 |
| SEBS E94 | Propylenglykol (5%) | 134 |
| SEBS E94 | 2-Octyldodecanol (5%) | 63 |
| SEBS E94 | Pollyethylenglykol(450)monododecylether (5%) | 153 |

Die homogene Verteilung des Wirkstoffes in der Heißschmelzklebemasse erfolgt in einem Thermohomogenisator wie zum Beispiel Thermomixer, Thermokneter, Walzenwerke oder Schneckensysteme. Die Zugabe des Wirkstoffs in die aufgeschmolzene Klebmasse erfolgt vorzugsweise in im Hilfsstoff gelöster Form, sie kann aber auch in reiner Form erfolgen.

**Patentansprüche**

1. Wirkstoffhaltige Pflaster enthaltend ein Trägermaterial und eine darauf zumindest partiell aufgebrachten Heißschmelzklebemasse, wobei die Heißschmelzklebemasse Ibuprofen sowie Hilfsstoffe enthält. **dadurch gekennzeichnet, dass** die Hilfsstoffe gewählt werden aus der Gruppe

   a.) der Ester gebildet aus Fettsäuren mit 8 bis 18 Kohlenstoffatomen und kurzkettigen Alkoholen oder Fettalkoholen und/oder Ester oder Ether gebildet aus Polyethylenglykol der Formel

$$H\left[O-CH_2-CH_2\right]_n OH$$

mit n= 6 bis 12 und Fettalkoholen mit 8 bis 18 Kohlenstoffatomen und/oder
b.) der Propylenglycol-mono-, di-ester, Glycerinmono-, -di- und -triester mit Fettalkoholen mit 8 bis 18 Kohlenstoffatomen, Fettalkoholen mit 8 bis 18 Kohlenstoffatomen, Propylenglykol und Polyethylenglykol.

**2.** Wirkstoffhaltige Pflaster gemäß Anspruch 1, **dadurch gekennzeichnet, dass** die Heißschmelzklebemasse Ibuprofen in einer Menge von 0,01 bis 50 Gew.-% enthält.

**3.** Wirkstoffhaltige Pflaster gemäß Anspruch 2, **dadurch gekennzeichnet, dass** die Heißschmelzklebemasse Ibuprofen in einer Menge von 0,1 bis 20 Gew.% enthält.

**4.** Wirkstoffhaltige Pflaster nach einem der vorstehenden Ansprüche, **dadurch gekennzeichnet, dass** die Hilfsstoffe in der Heißschmelzklebemasse zu 1 bis 20 Gew.% enthalten sind.

**5.** Wirkstoffhaltige Pflaster nach Anspruch 4, **dadurch gekennzeichnet, dass** die Hilfsstoffe in der Heißschmelzklebemasse zu 2 bis 15 Gew.% enthalten sind.

**6.** Wirkstoffhaltige Pflaster nach einem der vorstehenden Ansprüche, **dadurch gekennzeichnet, dass** die Heißschmelzklebemasse eine Polystyrol-Blockcopolymer-Heißschmelzklebemasse ist.

**7.** Wirkstoffhaltige Pflaster nach Anspruch 6, **dadurch gekennzeichnet, dass** die Heißschmelzklebemasse eine Polystyrolblock-copoly(ethylenbutylen)-block-Polystyrol-Heißschmelzklebemasse ist.

**8.** Verwendung von Hilfsstoffen zur Verbesserung der Freisetzung von Ibuprofen aus auf Trägermaterialien zumindest partiell aufgebrachten Heißschmelzklebemassen, **dadurch gekennzeichnet, dass** die Hilfsstoffe gewählt werden aus der Gruppe

a.) der Ester gebildet aus Fettsäuren mit 8 bis 18 Kohlenstoffatomen und kurzkettigen Alkoholen oder Fettalkoholen und/oder Ester oder Ether gebildet aus Polyethylenglykol der Formel

$$H\left[O-CH_2-CH_2\right]_n OH$$

mit n= 6 bis 12 und Fettalkoholen mit 8 bis 18 Kohlenstoffatomen und/oder
b.) der Propylenglycol-mono-, di-ester, Glycerinmono-, -di- und -triester mit Fettalkoholen mit 8 bis 18 Kohlenstoffatomen, Fettalkoholen mit 8 bis 18 Kohlenstaflatomen, Propylenglykol und Polyethylenglykol.

**9.** Verwendung von Hilfsstoffen gemäß Anspruch 8, **dadurch gekennzeichnet, dass** die Heißschmelzklebemasse Ibuprofen in einer Menge von 0,01 bis 50 Gew.-%enthält.

**10.** Verwendung von Hilfsstoffen gemäß Anspruch 9, **dadurch gekennzeichnet, dass** die Heißschmelzklebemasse Ibuprofen in einer Menge von 0,1 bis 20 Gew.-% enthält.

**11.** Verwendung von Hilfsstoffen nach einem der vorstehenden Ansprüche 8 bis 10, **dadurch gekennzeichnet, dass** die Hilfsstoffe in der Heißschmelzklebemasse zu 1 bis 20 Gew.-% enthalten sind.

**12.** Verwendung von Hilfsstoffen nach Anspruch 11, **dadurch gekennzeichnet, dass** die Hilfsstoffe in der Heißschmelzklebemasse zu 2 bis 15 Gew.-% enthalten sind.

**13.** Verwendung von Hilfsstoffen nach einem der vorstehenden Ansprüche 8 bis 12, **dadurch gekennzeichnet, dass** die Heißschmelzklebemasse eine Polystyrol-Blockcopolymer-Heißschmelzklebemasse ist.

**14.** Verwendung von Hilfsstoffen nach Anspruch 13, **dadurch gekennzeichnet, dass** die Heißschmelzklebemasse eine Polystyrolblock-copoly(ethylenbutylen)-block-Polystyrol-Heißschmelzklebemasse ist.

## Claims

**1.** Active ingredient-containing patches comprising a substrate material and, at least partially applied thereto, a hot melt adhesive composition, where the hot melt adhesive composition contains ibuprofen and auxiliaries, **characterized in that** the auxiliaries are chosen from the group

a.) of esters formed from fatty acids having 8 to 18 carbon atoms and short-chain alcohols or fatty alcohols and/or esters or ethers formed from polyethylene glycol of the formula

$$H{-}\left[O-CH_2-CH_2\right]_n{-}OH$$

with n = 6 to 12 and fatty alcohols having 8 to 18 carbon atoms and/or
b.) of the propylene glycol mono-, diesters, glycerol mono-, di- and triesters with fatty alcohols having 8 to 18 carbon atoms, fatty alcohols having 8 to 18 carbon atoms, propylene glycol and polyethylene glycol.

**2.** Active ingredient-containing patches according to Claim 1, **characterized in that** the hot melt adhesive composition contains ibuprofen in an amount of from 0.01 to 50% by weight.

**3.** Active ingredient-containing patches according to Claim 2, **characterized in that** the hot melt adhesive composition contains ibuprofen in an amount of from 0.1 to 20% by weight.

**4.** Active ingredient-containing patches according to any of the preceding claims, **characterized in that** the content of auxiliaries in the hot melt adhesive composition is from 1 to 20% by weight.

**5.** Active ingredient-containing patches according to Claim 4, **characterized in that** the content of auxiliaries in the hot melt adhesive composition is from 2 to 15% by weight.

**6.** Active ingredient-containing patches according to any of the preceding claims, **characterized in that** the hot melt adhesive composition is a polystyrene block copolymer hot melt adhesive composition.

**7.** Active ingredient-containing patches according to Claim 6, **characterized in that** the hot melt adhesive composition is a polystyrene block copoly(ethylenebutylene) block polystyrene hot melt adhesive composition.

**8.** Use of auxiliaries for improving the release of ibuprofen from hot melt adhesive compositions at least partially applied to substrate materials, **characterized in that** the auxiliaries are chosen from the group

a.) of esters formed from fatty acids having 8 to 18 carbon atoms and short-chain alcohols or fatty alcohols and/or esters or ethers formed from polyethylene glycol of the formula

$$H{-}\left[O-CH_2-CH_2\right]_n{-}OH$$

with n = 6 to 12 and fatty alcohols having 8 to 18 carbon atoms and/or
b.) of the propylene glycol mono-, diesters, glycerol mono-, di- and triesters with fatty alcohols having 8 to 18 carbon atoms, fatty alcohols having 8 to 18 carbon atoms, propylene glycol and polyethylene glycol.

**9.** Use of auxiliaries according to Claim 8, **characterized in that** the hot melt adhesive composition contains ibuprofen in an amount of from 0.01 to 50% by weight.

**10.** Use of auxiliaries according to Claim 9, **characterized in that** the hot melt adhesive composition contains ibuprofen

in an amount of from 0.1 to 20% by weight.

**11.** Use of auxiliaries according to any of the preceding Claims 8 to 10, **characterized in that** the content of auxiliaries in the hot melt adhesive composition is from 1 to 20% by weight.

**12.** Use of auxiliaries according to Claim 11, **characterized in that** the content of auxiliaries in the hot melt adhesive composition is from 2 to 15% by weight.

**13.** Use of auxiliaries according to any of preceding Claims 8 to 12, **characterized in that** the hot melt adhesive composition is a polystyrene block copolymer hot melt adhesive composition.

**14.** Use of auxiliaries according to Claim 13, **characterized in that** the hot melt adhesive composition is a polystyrene block copoly(ethylenebutylene) block polystyrene hot melt adhesive composition.

## Revendications

**1.** Pansement contenant une substance active, contenant un matériau support et une masse adhésive fondue à chaud appliquée au moins partiellement sur ledit matériau, la masse adhésive fondue à chaud contenant de l'Ibuprofen ainsi que des adjuvants, **caractérisé en ce que** les adjuvants sont choisis dans le groupe :

a) des esters formés à partir d'acides gras comprenant 8 à 18 atomes de carbone et d'alcools à courte chaîne ou d'alcools gras et/ou des esters ou des éthers formés à partir de polyéthylèneglycol de formule

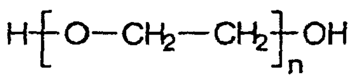

avec n=6 à 12 et d'alcools gras comprenant 8 à 18 atomes de carbone et/ou
b) des monoesters, des diesters de propylèneglycol, des monoesters, des diesters et des triesters de glycérol avec des alcools gras comprenant 8 à 18 atomes de carbone, des alcools gras comprenant 8 à 18 atomes de carbone, du propylèneglycol et du polyéthylèneglycol.

**2.** Pansement contenant une substance active selon la revendication 1, **caractérisé en ce que** la masse adhésive fondue à chaud contient l'Ibuprofen en une quantité de 0,01 à 50% en poids.

**3.** Pansement contenant une substance active selon la revendication 2, **caractérisé en ce que** la masse adhésive fondue à chaud contient l'Ibuprofen en une quantité de 0,1 à 20% en poids.

**4.** Pansement contenant une substance active selon l'une quelconque des revendications précédentes, **caractérisé en ce que** les adjuvants sont contenus dans la masse adhésive fondue à chaud à raison de 1 à 20% en poids.

**5.** Pansement contenant une substance active selon la revendication 4, **caractérisé en ce que** les adjuvants sont contenus dans la masse adhésive fondue à chaud à raison de 2 à 15% en poids.

**6.** Pansement contenant une substance active selon l'une quelconque des revendications précédentes, **caractérisé en ce que** la masse adhésive fondue à chaud est une masse adhésive fondue à chaud d'un copolymère à blocs de polystyrène.

**7.** Pansement contenant une substance active selon la revendication 6, **caractérisé en ce que** la masse adhésive fondue à chaud est une masse adhésive fondue à chaud de bloc de polystyrène-copoly(éthylènebutylène)-bloc de polystyrène.

**8.** Utilisation d'adjuvants pour améliorer la libération d'Ibuprofen de masses adhésives fondues à chaud appliquées au moins partiellement sur des matériaux support, **caractérisée en ce que** les adjuvants sont choisis dans le groupe

a) des esters formés à partir d'acides gras comprenant 8 à 18 atomes de carbone et des alcools à courte chaîne ou des alcools gras et/ou des esters ou des éthers formés à partir de polyéthylèneglycol de formule

$$H{-}[O{-}CH_2{-}CH_2]_n{-}OH$$

avec n=6 à 12 et d'alcools gras comprenant 8 à 18 atomes de carbone et/ou

b) des monoesters, des diesters de propylèneglycol, des monoesters, des diesters et des triesters de glycérol avec des alcools gras comprenant 8 à 18 atomes de carbone, des alcools gras comprenant 8 à 18 atomes de carbone, du propylèneglycol et du polyéthylèneglycol.

**9.** Utilisation d'adjuvants selon la revendication 8, **caractérisée en ce que** la masse adhésive fondue à chaud contient l'Ibuprofen en une quantité de 0,01 à 50% en poids.

**10.** Utilisation d'adjuvants selon la revendication 9, **caractérisée en ce que** la masse adhésive fondue à chaud contient l'Ibuprofen en une quantité de 0,1 à 20% en poids.

**11.** Utilisation d'adjuvants selon l'une quelconque des revendications précédentes 8 à 10, **caractérisée en ce que** les adjuvants sont contenus dans la masse adhésive fondue à chaud à raison de 1 à 20% en poids.

**12.** Utilisation d'adjuvants selon la revendication 11, **caractérisée en ce que** les adjuvants sont contenus dans la masse adhésive fondue à chaud à raison de 2 à 15% en poids.

**13.** Utilisation d'adjuvants selon l'une quelconque des revendications précédentes 8 à 12, **caractérisée en ce que** la masse adhésive fondue à chaud est une masse adhésive fondue à chaud d'un copolymère à blocs de polystyrène.

**14.** Utilisation d'adjuvants selon la revendication 13, **caractérisée en ce que** la masse adhésive fondue à chaud est une masse adhésive fondue à chaud de bloc de polystyrène-copoly (éthylènebutylène)-bloc de polystyrène.